(19) Europäisches Patentamt
European Patent Office
Office européen des brevets

(11) **EP 2 676 661 B1**

(12) **EUROPEAN PATENT SPECIFICATION**

(45) Date of publication and mention
of the grant of the patent:
**22.03.2017 Bulletin 2017/12**

(21) Application number: **12747874.1**

(22) Date of filing: **14.02.2012**

(51) Int Cl.:
*A61K 9/24* *(2006.01)*      *A61K 9/28* *(2006.01)*
*A61K 9/22* *(2006.01)*      *A61K 47/38* *(2006.01)*
*A61K 31/18* *(2006.01)*      *A61K 9/20* *(2006.01)*

(86) International application number:
**PCT/KR2012/001116**

(87) International publication number:
**WO 2012/111965 (23.08.2012 Gazette 2012/34)**

(54) **ORALLY ADMINISTERED SUSTAINED-RELEASE TRIPLE-LAYER PILL CONTAINING TAMSULOSIN OR PHARMACEUTICALLY ACCEPTABLE SALT THEREOF**

ORAL VERABREICHTE DREILAGIGE TABLETTE MIT VERZÖGERTER FREISETZUNG MIT TAMSULOSIN ODER EINEM PHARMAZEUTISCH UNBEDENKLICHEN SALZ DAVON

COMPRIMÉ À TROIS COUCHES À LIBÉRATION PROLONGÉE, ADMINISTRÉ PAR VOIE ORALE, CONTENANT DE LA TAMSULOSINE OU UN SEL PHARMACEUTIQUEMENT ACCEPTABLE DE CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.02.2011 KR 20110013099**

(43) Date of publication of application:
**25.12.2013 Bulletin 2013/52**

(73) Proprietor: **GL Pharmtech Co., Ltd.
Seongnam, Gyeonggi 462-807 (KR)**

(72) Inventors:
• **PARK, Jung Soo
Seongnam-si
Gyeonggi-do 463-030 (KR)**
• **SHIM, Ji Yeon
Seongnam-si
Gyeonggi-do 463-070 (KR)**
• **PARK, Jun Sang
Yongin-si
Gyeonggi-do 448-744 (KR)**

(74) Representative: **Scholz, Volker
Boehmert & Boehmert
Anwaltspartnerschaft mbB
Patentanwälte Rechtsanwälte
Pettenkoferstrasse 20-22
80336 München (DE)**

(56) References cited:
WO-A1-01/30322          KR-A- 20040 045 148
KR-A- 20080 073 989     US-A1- 2007 270 459
US-A1- 2010 040 681     US-B1- 6 861 070

**Description**

[Technical Field]

The present invention relates to a sustained-release triple-layer tablet for oral administration including tamsulosin or a pharmaceutically acceptable salt thereof (hereinafter also collectively referred to as tamsulosin). More specifically, the present invention relates to a formulation that is structurally separated and includes an intermediate layer containing water soluble excipients to absorb a large amount of water in a rapid and continuous manner compared to matrix formulations composed of only swellable polymers, enabling release of the drug even in the lower digestive tract including the colon.

[Background Art]

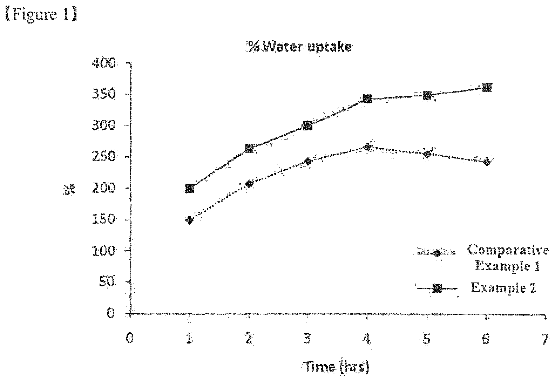

Korean Patent Registration No. 10-0355130 discloses a sustained-release hydrogel preparation produced by mixing a hydrophilic excipient with a matrix tablet based on PEO as a swellable polymer.
Korean Patent Publication No. 10-2005-0107298 discloses a process for producing a medicinal composition by spraying a PEG suspension on a PEO powder using a fluidized bed coater to prepare a tablet, followed by compressive molding.
Korean Patent Registration No. 10-0507400 provides a method for preventing release properties of medicinal compositions despite storage under light irradiation by mixing or coating a matrix tablet composed of PEO with iron oxide.
Korean Patent Registration No. 10-0582350 discloses a highly stable tamsulosin hydrochloride composition for oral administration including tamsulosin hydrochloride, polyvinyl acetate and water soluble hydroxypropyl methylcellulose, and a controlled-release granule formulation thereof.
Document US 2010/0040681 discloses triple-layered sustained release tamsulosin hydrochloride tablet (table 3, figure 1, figure 4). Intermediate layer contains tamsulosin, povidone, copovidone, dextrate and lactose. The external layers contain PEO and magnesium stearate.

[Disclosure]

[Technical Problem]

Novel formulations should meet the guidelines adopted by the International Conference on Harmonization of technical requirements for registration of pharmaceuticals for human use (ICH) for commercialization. The guidelines were developed for the purpose of standardizing related regulations for the approval of pharmaceuticals in different areas. According to the description of ICH Q3B (R2), in the case of a new drug whose daily maximum dosage is 1 mg, individual degradation products having undetermined structures should be less than 0.1% or less and individual degradation products having determined structures should be less than 1.0%. These guidelines are the same throughout the world. New drugs cannot be commercialized until degradation products comply with the common guidelines. The Provision for Specifications and Test Procedures of Drugs (The Korea Food and Drug Administration Notification No. 2007-47, revised on July 2, 2007) adopts guidelines for the determination of drug contents.
According to content specifications, drug substances should be set to more than 99.0%, single active ingredient preparations should be set to 95.0-105.0%, and combined active ingredients preparations should be set to 90.0-110.0%. However, they shall be differently established if there are permitted specifications from the manufacturing country or the original country or there is a proper rationale set as a specification value necessary for guaranteeing stability and effectiveness based on production procedure, quantitative error, stability, etc. In the case where the rationale data is test data, the content specifications can be set for test materials of at least 3 lots taking into consideration actual statistical values obtained by testing every lot at 3 times or more. Since the present invention is intended to provide a sustained-release formulation containing tamsulosin as a single active ingredient, the content of tamsulosin per tablet should be in the range of 95.0% to 105.0%, individual degradation products having undetermined structures should be less than 0.1% or less, and individual degradation products having determined structures should be less than 1.0%. Within these ranges, the formulation can be prepared for commercialization. Under these circumstances, the present inventors have found that when a composition is compounded with tamsulosin, no degradation products of the drug and no reduction in the content of the drug are observed, which are advantageous in terms of the stability of the formulation. Based on this finding, the present inventors arrived at the present invention.
The inventors of the present invention have conducted in-depth research on sustained-release formulations using swellable polymers, and as a result, found that a formulation should contain the largest possible amount of water during staying in the upper digestive tract such as the small intestine in order to release a drug even in the colon where a small amount of water is present. The present inventors have also found that when a tablet is designed to have a triple layer structure in which upper and lower layers contain sustained-release bases and an intermediate layer contains a hydrophilic substance or unswellable excipients, it has a higher water uptake than a matrix tablet prepared from a simple mixture of a swellable polymer and a hydrophilic excipient.

However, polyethylene oxide as a sustained-release polymer used in the present invention undergoes a reduction in molecular weight depending on what other excipients the polymer is compounded with. Autooxidation of the polymer promotes a reduction in molecular weight, and as a result, the polymer will gradually lose its role as a sustained-release base. Particularly, polyethylene oxide is incompatible with a strong oxidant (Handbook of Pharmaceutical excipients, 4th). In CRS, 2008, DOW reported that when lactose or mannitol is compounded with polyethylene oxide to prepare a tablet, the dissolution behavior of a drug is changed after the shelf life of the tablet. That is, the excipient is incompatible with polyethylene oxide. This report also describes that the influence of the reducing sugar such as lactose or mannitol on the molecular weight reduction of polyethylene oxide is because autooxidation promoted by air causes degradation the polymer into monomers. As a consequence, the viscosity of the polyethylene oxide degraded is reduced in a medium, and therefore, the polymer loses its role as a sustained-release base for the sustained-release tablet stored under storage conditions for stability, causing an increased dissolution of the drug. However, the tamsulosin sustained-release tablet of the present invention is divided into three layers. Particularly, since polyethylene oxide incompatible with mannitol is compounded in a rapid-release layer containing the drug, degradation of the sustained-release polymer into monomers can be minimized through the interlayer separation of polyethylene oxide from mannitol, leading to the formation of few degradation products while ensuring stability of the drug in terms of dissolution behavior. In addition, the present inventors have found that the stability of the tamsulosin sustained-release tablet may greatly vary depending on the physicochemical properties of excipients compounded in the tablet, and the use of mannitol or polyethylene glycol leads to an insignificant interaction between tamsulosin as the drug and polyethylene oxide as the sustained-release base, causes no problems during preparation, induces negligible loss of efficacy of tamsulosin according to storage conditions, and results in the formation of degradation products at a level complying with the guidelines adopted by the ICH. The present invention has been accomplished based on this finding.

[Technical Solution]

The present invention provides a tamsulosin sustained-release triple-layer tablet for oral administration as defined in claims. The stability of the tamsulosin sustained-release triple-layer tablet may vary depending on which layer contains tamsulosin in view of the structural characteristics of the tablet including three layers coupled to each other, unlike matrix tablets. Since an active ingredient is uniformly distributed in a matrix tablet, the compatibility of the active ingredient with all excipients compounded in the tablet needs to be confirmed. In the case of a triple-layer tablet, however, the compatibility of an active ingredient with all excipients to be compounded in a layer containing the active ingredient should be preferentially ensured. Another requirement is that the influence of the excipients compounded with the active ingredient on polyethylene oxide as a sustained-release base should be minimized. Poor compatibility between tamsulosin and the excipients causes a reduction in the content of the active ingredient and an increase in the amount of degradation products with the passage of time although there is a difference depending on storage conditions. The present inventors have made efforts in order to solve the above problems, and as a result, discovered that when D-mannitol, polyethylene glycol and electrolytes are used alone or as a mixture with other hydrophilic excipients in a drug-containing intermediate layer of a tamsulosin sustained-release triple-layer tablet, no change in compatibility with the excipients is caused.

Therefore, the present invention is characterized in that compatibility between excipients and tamsulosin in the intermediate layer is ensured to control the release of the active ingredient in a continuous manner without interlayer separation of the triple-layer tablet and losing activity of the active ingredient.

The tamsulosin sustained-release triple-layer tablet of the present invention will now be described in more detail.

[Advantageous Effects]

The tamsulosin sustained-release triple-layer tablet for oral administration according to the present invention is designed such that tamsulosin can be easily absorbed even in the human gastrointestinal tract where a small amount of water is present. Excipients compounded in the intermediate layer containing tamsulosin cause less increase in the amount of degradation products and less reduction in the content of tamsulosin, resulting in a marked improvement in the stability of the triple-layer tablet. In addition, the release of tamsulosin is effectively controlled regardless of storage conditions, so that the effective blood concentration of tamsulosin can be advantageously maintained constant for a long time.

[Description of Drawings]

FIG. 1 shows the water uptakes of tablets prepared in Example 2 and Comparative Example 1.

[Best Mode]

The triple-layer tablet of the present invention includes a rapid-release intermediate layer. The intermediate layer is present in an amount of 5 to 60% by weight, preferably 10 to 40% by weight, based on the total weight of the tablet.
The amount of tamsulosin in the rapid-release intermediate layer may be from 0.1 to 60% by weight, based on the total weight of the tablet.
The composition of the rapid-release intermediate layer uses one or more pharmaceutically acceptable water soluble excipients selected from mannitol and polyethylene glycol.
Each of the upper and lower layers contains a swellable polymer. The upper and lower layers are present in an amount of 40 to 95% by weight, preferably 60 to 90% by weight, based on the total amount of the tablet. The composition and amount of the upper layer may be the same as those of the lower layer. Alternatively, the upper and lower layers may be designed to have different compositions and amounts.
The swellable polymer is polyethylene oxide. The polymer is preferably polyethylene oxide having a viscosity of 400 cps or more (2% aqueous solution).
Tamsulosin may be optionally added to the upper and lower layers as well as the rapid-release intermediate layer for the purpose of ensuring various release profiles.
Each of the upper and lower layers may further include at least one additive. Examples of such additives include: pharmaceutically acceptable excipients, such as lactose, dextrose, sucrose, dextrate, mannitol, sorbitol, xylitol, sodium chloride, potassium chloride, magnesium chloride, calcium hydrogen phosphate, citric acid, and microcrystalline cellulose; binders, such as copovidone, polyvinylpyrrolidone, and hydroxypropyl cellulose; and lubricants, such as magnesium stearate, stearic acid, sodium stearyl fumarate, and light anhydrous silicic acid.
The intermediate layer includes tamsulosin and is essentially composed of hydrophilic excipients. The intermediate layer serves to allow the tablet to have a constant dissolution behavior irrespective of the solubility of other main ingredients. The use of the excipients in the intermediate layer contributes to a higher water uptake of the tablet than simple sustained-release matrix tablets.
On the other hand, the present invention provides a method for preparing the tamsulosin sustained-release triple-layer tablet for oral administration. The method includes: mixing a pharmacologically active substance with pharmaceutically allowable additives and optionally granulating the mixture to produce tablet materials, which are to be distributed in the rapid-release intermediate layer; mixing a swellable polymer, a pharmaceutically allowable additive and optionally a pharmacologically active substance, and optionally granulating the mixture to produce tablet materials, which are to be distributed in the upper and lower layers; and sequentially compressing the mixtures.
The tablet materials may be produced by general methods. Specifically, the sustained-release triple-layer tablet for oral administration is prepared by mixing the constituent ingredients of the respective layers using a mixer, and directly compressing the mixtures using a multilayer tablet press. Alternatively, the sustained-release triple-layer tablet may be prepared by mixing the constituent ingredients of the respective layers, granulating the mixtures using a mechanical device such as a vertical granulator or a roller compressor, and compressing the granules using a multilayer tablet press.
Hereinafter, the present invention will be explained in more detail with reference to the following examples. However, these examples are provided for illustrative purposes only and are not intended to limit the scope of the invention.

[Mode for Invention]

Example 1

As shown in Table 1, tamsulosin hydrochloride as an active ingredient was mixed with the excipient in a ratio of 1:10 taking into consideration the amount of the drug present per tablet. The compatibility of the tamsulosin hydrochloride with the excipient was confirmed with time. Table 1 shows changes in the content of the tamsulosin hydrochloride under extreme test conditions during the test period.

Extreme test conditions

① Temperature 40 °C, relative humidity 75%

② Packaging condition: Not packaged
③ Test period: 2 weeks

[Table 1]

| No. | Active ingredient | Excipient | 0 d | 3 d | 7 d | 14d | Changes in state and appearance |
|-----|-------------------|-----------|-----|-----|-----|-----|-------------------------------|
| 1 | Tamsulosin hydrochloride | - | 100.0 | 100.0 | 95.0 | 93.5 | White |
| 2 | Tamsulosin hydrochloride | Dextrate monohydrate | 100.0 | 87.4 | 86.0 | 85.5 | Brown |
| 3 | Tamsulosin hydrochloride | D-mannitol | 100.0 | 100.0 | 100.0 | 98.3 | White |
| 4 | Tamsulosin hydrochloride | Polyethylene glycol 6,000 | 100.0 | 100.0 | 100.0 | 98.4 | White |
| 5 | Tamsulosin hydrochloride | D-sorbitol | 100.0 | 53.0 | - | - | Dissolved |
| 6 | Tamsulosin hydrochloride | Anhydrous lactose | 100.0 | 100.0 | 100.0 | 94.0 | Light brown |
| 7 | Tamsulosin hydrochloride | Microcrystalline cellulose | 100.0 | 100.0 | 98.3 | 98.2 | Light brown |
| 8 | Tamsulosin hydrochloride | Maltodextrin | 100.0 | 86.4 | 85.2 | 84.0 | White |
| 9 | Tamsulosin hydrochloride | Maltose | 100.0 | 100.0 | 92.1 | 90.0 | White |

According to the test results in Table 1, D-mannitol and polyethylene glycol 6,000 are expected to be soluble in water, undergo no changes in state and appearance, and cause less reduction in the content of tamsulosin hydrochloride or less increase in the amount of degradation products when tablets are prepared by compounding with tamsulosin hydrochloride, followed by packaging. It is noted that the content of tamsulosin hydrochloride was slowly reduced when tamsulosin hydrochloride was compounded with mannitol or polyethylene glycol 6,000, compared to when tamsulosin hydrochloride was stored alone. Therefore, the use of mannitol or polyethylene glycol 6,000 is expected to effectively stabilize tamsulosin hydrochloride. In view of these results, the two excipients without undergoing changes in state and appearance and causing less content reduction were used as essential ingredients to constitute an intermediate layer.

Example 2

As shown in Table 2, the same amount and kind of the swellable polymer was mixed with the lubricant and the mixture was used without additional sieving to form upper and lower layers. The available excipients and tamsulosin hydrochloride were passed through a 50-mesh sieve and mixed. Finally, magnesium stearate was added without additional sieving. The resulting mixture was used to form an intermediate layer. After packaging with a PTP aluminum foil, changes in the content of the tamsulosin hydrochloride and the amount of degradation products were observed under the following storage conditions. Table 2 shows the composition (unit: mg) of the tablet of Example 2 and Table 4 shows changes in the amount of degradation products.

Storage conditions

① Accelerated conditions: Temperature 40 °C, relative humidity 75%
② Long-term storage conditions: Temperature 25 °C, relative humidity 60%
③ Test period: 6 months
④ Packaging material: PTP aluminum foil

[TABLE 2]

| Layer | Ingredient | Example 2 |
|---|---|---|
| Upper layer | Polyethylene oxide (Polyox WSR 303 NF) | 100.00 |
| | Magnesium stearate | 0.50 |
| Intermediate layer | Tamsulosin hydrochloride | 0.40 |
| | D-mannitol | 30.00 |
| | Polyethylene glycol 6000 | 20.00 |
| | Sodium chloride | 9.50 |
| | Magnesium stearate | 0.50 |
| Lower layer | Polyethylene oxide (Polyox WSR 303 NF) | 100.0 |
| | Magnesium stearate | 0.50 |
| | Total | 261.40 |

[TABLE 3]

| Example 2 | | |
|---|---|---|
| | Temperature 40 °C, Relative humidity 75% | Temperature 25 °C, Relative humidity 60% |
| Month | Mean (%) | Mean (%) |
| 0 month | 100.0 | 100.0 |
| 3 month | 97.7 | 99.4 |
| 6 month | 96.5 | 102.0 |

[TABLE 4]

| Example 2 | | |
|---|---|---|
| | Temperature 40 °C, Relative humidity 75% | Temperature 25 °C, Relative humidity 60% |
| Month | Mean (%) | Mean (%) |
| 0 month | 0.02 | 0.02 |
| 3 month | 0.05 | 0.04 |
| 6 month | 0.08 | 0.04 |

As can be seen from the results in Tables 3 and 4, when D-mannitol as an excipient, polyethylene glycol 6000 as a binder, and sodium chloride as a dissolution assistant were used in Example 2, no changes in state and appearance, less reduction in the content of tamsulosin hydrochloride, and less increase in the amount of degradation products were observed. These results are based on the results shown in Example 1 and confirm that the long-term stability of the tablet was ensured, leading to an improvement in storage stability and the triple-layer tablet including the intermediate layer containing tamsulosin hydrochloride is suitable for practical use.

Comparative Example 1

The ingredients were mixed in the amounts (unit: mg) shown in Table 5. The mixture was passed through a 30-mesh sieve to prepare a tablet material. The tablet material was filled in a die having a diameter of 9.0 mm and compressed at a pressure of 6 MPas using a hydraulic press to prepare a tablet.

[TABLE 5]

| Ingredient | Comparative Example 1 |
|---|---|
| Polyethylene oxide (Polyox WSR 303 NF) | 200.0 |
| Polyethylene glycol (Macrogol 6000) | 40.0 |
| Magnesium stearate | 0.5 |
| Total | 240.5 |

Measurement of time-dependent water uptakes of tablets

900 mL of the second disintegration test solution according to the Korean Pharmacopoeia is put in a 1 L beaker. Each of the tablets and the disintegration test solution were stirred at about 500 rpm with a 2.5 cm long magnetic bar in Mag-Mixer MG600 (Yamato). Every one hour from 1 to 6 hrs after initiation of the test, the gelled tablet was taken out of the mixer and weighed to calculate an increment in weight compared to the initial weight of the tablet.

$$* \text{ Water uptake (\%)} = \frac{W_t - W_I}{W_I} \times 100$$

$W_T$: Weight of the gelled tablet after T h
$W_I$: Initial weight of the tablet before gelling test

The test results are shown in Fig. 1. In Comparative Example 1 and Example 2, the swellable polymers were used in the same amount of about 200 mg. The water uptakes of the tablets were different by a minimum of about 50% and a maximum of about 100% with time due to their structural difference. Further, the water uptake of the tablet of Comparative Example 1 showed a tendency to decrease after 4 hrs due to the saturation of the swellable polymer, whereas the water uptake of the tablet of Example 2 contrast, in Example 1 steadily increased for 6 hrs due to the presence of the hydrophilic excipients in the intermediate layer.

**Claims**

1.  An oral sustained-release triple-layer tablet comprising:

    (1) a rapid-release intermediate layer containing tamsulosin or a pharmaceutically acceptable salt thereof and at least one water soluble excipient selected from the group consisting of mannitol and polyethylene glycol; and
    (2) upper and lower layers, each of which contains polyethylene oxide, wherein the polyethylene oxide is a swellable polymer,

    wherein the upper and lower layers swell and surround exposed sides of the rapid-release intermediate layer upon contact with a water soluble medium to control the release of the active substance.

2.  The tablet according to Claim 1, wherein the upper and lower layers further comprise tamsulosin or a pharmaceutical salt thereof.

3.  The tablet according to Claim 1, wherein the water uptake of the tablet is steadily increased for 6 hours in pH 6.8 buffer of second disintegration test solution according to Korean Pharmacopoeia.

4.  The tablet according to Claim 3, wherein the tablet can also release tamsulosin at colon.

**Patentansprüche**

1.  Orale dreilagige Tablette mit verzögerter Freisetzung, umfassend:

**EP 2 676 661 B1**

(1) eine schnell freisetzende Zwischenlage enthaltend Tamsulosin oder ein pharmazeutisch annehmbares Salz desselben und wenigstens einen wasserlöslichen Arzneiträger ausgewählt aus der Gruppe bestehend aus Mannitol und Polyethylenglykol; und

(2) obere und untere Lagen, wobei jede derselben Polyethylenoxid enthält, wobei das Polyethylenoxid ein quellbares Polymer ist,

wobei die oberen und unteren Lagen exponierte Seiten der schnell freisetzenden Zwischenlage bei Kontakt mit einem wasserlöslichen Medium quellen und umgeben, um die Freisetzung der aktiven Substanz zu steuern.

2. Tablette nach Anspruch 1, wobei die oberen und unteren Lagen ferner Tamsulosin oder ein pharmazeutisches Salz desselben umfassen.

3. Tablette nach Anspruch 1, wobei die Wasseraufnahme der Tablette stetig für sechs Stunden bei pH-Pufferung von 6,8 einer zweiten Disintegrationstestlösung gemäß des koreanischen Arzneimittelbuches erhöht wird.

4. Tablette nach Anspruch 3, wobei die Tablette ebenfalls Tamsulosin im Dickdarm freisetzen kann.


**Revendications**

1. Comprimé à libération prolongée et à trois couches administré par voie orale comprenant :

(1) une couche intermédiaire à libération rapide contenant de la tamsulosine ou un sel pharmaceutiquement acceptable de celle-ci et au moins un excipient hydrosoluble sélectionné dans le groupe consistant en le mannitol et le polyéthylène glycol ; et

(2) des couches externe et interne qui contiennent chacune un poly(oxyde d'éthylène), où le poly(oxyde d'éthylène) est un polymère gonflable,

où les couches externe et interne gonflent et entourent les faces exposées de la couche intermédiaire à libération rapide au contact avec un milieu hydrosoluble pour contrôler la libération du principe actif.

2. Comprimé selon la revendication 1, où les couches externe et interne comprennent également de la tamsulosine ou un sel pharmaceutique de celle-ci.

3. Comprimé selon la revendication 1, où la captation d'eau par le comprimé est augmentée de manière continue pendant 6 heures dans un tampon à pH 6,8 utilisé comme solution dans un deuxième test de désintégration réalisé conformément à la Pharmacopée coréenne.

4. Comprimé selon la revendication 3, où le comprimé peut également libérer la tamsulosine au niveau du côlon.

【Figure 1】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 100355130
- KR 1020050107298
- KR 100507400
- KR 100582350
- US 20100040681 A

### Non-patent literature cited in the description

- *The Korea Food and Drug Administration Notification No. 2007-47,* 02 July 2007